# EUROPEAN PATENT APPLICATION

(11) **EP 1 544 768 A1**
(43) Date of publication of application: **22.06.2005**
(21) Application number: 03795297.5
(22) Date of filing: 05.09.2003
(51) Int. Cl.: G06F 17/60

(54) **MEDICAL INFORMATION MANAGEMENT SYSTEM**

(30) Priority: 13.09.2002 JP 2002268710
(71) Applicant: KONICA MINOLTA HOLDINGS, INC., Tokyo 100-0005 (JP)
(72) Inventor: TAKEYAMA, Toshihisa, Hino-shi, Tokyo 191-8511 (JP)
(74) Representative: Henkel, Feiler & Hänzel
(86) International application number: PCT/JP2003/011356
(87) International publication number: WO 2004/025530

(57) **Abstract**

A medical information management system (100) including: plural database devices (2 to 8) for storing medical information in a database; a management device (1a) for managing the medical information integrally; and an operation terminal (A to E, 20 to 80) for operating at least one of the database devices (A to E) and management devices (1a). The database devices (2 to 8) have an automatic transmission section (21 to 61) for extracting necessary information from the stored medical information and sending the necessary information to the management device (1a). The management device (1a) includes a reception section (15) for receiving the medical information sent from the automatic transmission section (21 to 61); a storage section (16) for storing integrally the medical information received by the reception section (15) based on predetermined information included in the medical information; an individual verification section (12) for verifying whether an operator of the operation terminal (A to E, 20 to 80) is an individual registered beforehand; and a data access authentication section (13) for judging whether medical information instructed to be operated by the operation terminal (A to E, 20 to 80) is information capable of being operated.

## Description

### Technical Field

The present invention relates to a medical information management system.

### Background Art

In recent years, efficient medical care has been required, and networking and systematization are progressing inside and outside hospitals. There is a growing demand for total systems in which a hospital information system (HIS), a radiation department information system (RIS), a picture archiving and communications system (PACS), electronic charts, remote medical care and the like are linked organically. Moreover, medical institutions have gradually come to share data for the purpose of efficiently providing local medical care. This avoids redundant tests, treatments, and medication on patients.

Incidentally, when various pieces of data are connected by a network, the problem of ensuring security for preventing the leakage of personal information relating to patients, the problem of preventing falsification of the various pieces of data kept as digital data, and the like may arise.

Digital signatures, electronic signatures, or the like are adopted in electronic commerce and the like. In Japan, the "Law Relating to Electronic Signatures and Authentication Services" was enacted. In the United States, the "Electronic Signatures in Global and National Commerce Act" was enacted. In Europe, the "Directive on a Community Framework for Electronic Signatures" was issued. That is to say, an effort to specify persons has been made throughout the world. In addition, on the Internet, for example, the World-Wide Web Consortium (W3C), being a Web standardization body, proposed recommended standards for an XML-based electronic signature technique.

In the medical field, the introduction of a means of authenticating a patient by comparing fingerprints (see the patent documents 1 and 2, for example), authenticating a visitor to a medical institution, a person who serves in a medical institution, or a neonate who was born in a medical institution by comparing irises (see the patent documents 3 and 4, for example), authenticating a patient by comparing voiceprints (see the patent documents 5 and 6, for example), or the like has been proposed. In addition, attempts to limit in advance a medical staff who can access each of the above pieces of data have been made (see the patent documents 7-9, for example).

### Patent Documents

1. Japanese Patent Laid-Open Publication No. 2000-155782
2. Japanese Patent Laid-Open Publication No. 2001-312566
3. Japanese Patent Laid-Open Publication No. 2000-242788
4. Japanese Patent Laid-Open Publication No. 2001-76072
5. Japanese Patent Laid-Open Publication No. Hei 8-71148
6. Japanese Patent Laid-Open Publication No. Hei 10-201827
7. Japanese Patent Laid-Open Publication No. 2002-41656
8. Japanese Patent Laid-Open Publication No. 2002-82839
9. Japanese Patent Laid-Open Publication No. 2002-183319

However, when medical information is networked and is used in a wide area via the Internet, problems, such as medical malpractice and invasion of personal privacy caused by leakage of information, and the like will cause. Therefore, there is a growing demand for security from various viewpoints.

### Disclosure of The Invention

The present invention was made in view of the above-described problems. An object of the present invention is to provide a medical information management system considering security in response to a databases storing various medical information. Particularly, the object of the present invention is to provide a medical information system for managing medical information provided on the basis of individual (personal) information identified by an individual verification section.

In order to solve the above-described object, according to an aspect of the present invention, the medical information management system of the present invention comprises: a plurality of database devices for storing medical information in a database; a management device for managing the medical information integrally; and an operation terminal for operating at least one of the database devices and the management device, each of the database devices comprising an automatic transmission section for extracting necessary information from the medical information stored in the database and sending the necessary information to the management device, the management device comprising: a reception section for receiving the medical information sent from the automatic transmission section; a storage section for storing integrally the medical information received by the reception section on a basis of predetermined information included in the medical information; an individual verification section for verifying whether or not an operator of the operation terminal is an individual registered beforehand; and a data access authentication section for judging whether or not medical information instructed to be operated by the operation terminal is information capable of being operated.

According to the medical information management system of the present invention, in the medical information management system in which the medical information is managed integrally, verification of the operator for operating the medical information is performed, and moreover, the medical information to be operated by the verified operator is authenticated whether or not it is the information capable of being operated. Thereby, the medical information can be prevented from being operated improperly, and the security of information management can be improved. Further, when outputting the medical information, since it is outputted after authenticating whether the medical information instructed to be outputted is the information capable of being outputted, it becomes possible to prevent the important medical information from being leaked to outsiders.

In the medical information management system, preferably, the management device further comprises a control section for performing at least one of browsing, adding, modifying and processing of the medical information in case that the medical information instructed to be operated is judged as the information capable of being operated by the data access authentication section on the basis of operation instruction from the operator verified by the individual verification section.

Further, the system may comprise an output device for recording the medical information in a recording medium as electronic information or outputting the medical information in a printing medium as a hardcopy, and the management device may further comprise an output permission section for judging whether or not medical information instructed to be outputted by the operation terminal is information capable of being outputted to the output device and for outputting the medical information to the output device in case that the medical information is the information capable of being outputted.

Further, preferably, the management device further comprises a data falsification prevention section for storing change history in the storage section by making the change history correlate with the medical information in case that the adding, modifying or processing is performed to the medical information store in the storage section by the control section.

Thereby, when the operation of adding, modifying, processing or the like is performed to the medical information, since the change history is stored by being correlated with the medical information while maintaining the original medical information, it becomes possible to prevent the important medical information from being rewritten mistakenly or to prevent the medical information from being falsified improperly. Further, since the stored date and time or the changed date and time of the medical information is provided as attached information, an effect of inhibiting actions of operating the information improperly can be obtained.

Further, preferably, the management device further comprises a date and time providing section for adding a date and time when the medical information is operated as attached information in case that the browsing, adding, modifying or processing is performed to the medical information stored in the storage section by the control section. In this case, preferably, the date and time providing section adds the date and time when the medical information sent by the automatic transmission section is received by the reception section or the date and time when the medical information sent by the automatic transmission section is stored in the storage section to the medical information as the attached information.

Moreover, the medical information for being browsed, added, modified or processed by the control section is preferable to be at least one kind of data selected from character data, on-off data, static image data and dynamic image data.

Further, preferably, the storage section of the management device comprises a plurality of databases for storing information in accordance with a kind of the medical information, and the control section reads out the corresponding medical information from the plurality of the databases in accordance with the operation instruction from the operation terminal, and controls at least one operation of the browsing, adding, modifying and processing.

Moreover, in the medical information management system, preferably, the automatic transmission section detects difference between medical information to be sent and medical information sent in past, extracts medical information corresponding to the difference, and sends the extracted medical information to the management device as the necessary information. Further, the medical information sent by the automatic transmission section may include a creation date and time when the medical information is created, and the management device may further comprise a storage control section for comparing a creation date and time included in the medical information received by the reception section with a creation date and time included in the medical information stored in the storage section, and for storing the medical information received by the reception section, which includes the creation date and time different from the creation date and time included in the medical information stored in the storage section.

Preferably, the individual verification section verifies the individual according to at least one kind of individual information selected from a password, an ID card, a fingerprint, a palm print, a voiceprint, a face, a signature handwriting, an iris pattern, a eyeground pattern and a vein pattern. More preferably, the individual information is at least one of the fingerprint, the palm print, the voiceprint, the face, the iris pattern, the eyeground pattern and the vein pattern.

The individual verification section may verify the individual in combination with the individual information of the password or the ID card and at least one of the fingerprint, the voiceprint, the iris pattern, and the vein pattern.

Further, preferably, the operation terminal comprises an input member for inputting the operation instruction, and the input member is preferable to be at least one kind of member selected from a pen, a keyboard, a mouse and voice.

Further, preferably, the medical information includes at least one kind of data selected from clinical examination management system data, radiation department system data, hospital information system data, electronic chart system data, case management system data, medicine history management system data, medicine document data, nursing-care insurance system data, medical-related document data. Further, preferably, the medical information sent by the automatic transmission section includes at least one kind of data selected from clinical examination management system data, radiation department system data, hospital information system data, electronic chart system data, case management system data, medicine history management system data, medicine document data, nursing-care insurance system data, medical-related document data.

In addition, preferably, the management device and the operation terminal are connected via a network, and the network comprises a dedicated line for connecting the management device and the operation terminal.

Further, preferably, the management device further comprises: an encryption transmission section for encrypting and sending information sending to the operation terminal; and a decryption reception section for receiving and decrypting information sent from the operation terminal, and the operation terminal comprises: an encryption transmission section for encrypting and sending the information sending to the management device; and a decryption reception section for receiving and decrypting the information sent from the management device.

Further, according to another aspect of the present invention, the medical information management system of the present invention comprises: at least one database for storing medical information; and a management section for managing access to the database, wherein the management section stores a biological discrimination pattern peculiar to an operator who accesses the database by making the biological discrimination pattern correlate with ID information set to the operator, obtains a biological discrimination pattern and ID information from an arbitrary operator when an access is required to the database from the arbitrary operator, and judges whether or not to approve the access by comparing the obtained biological discrimination pattern and the ID information with the biological discrimination pattern and the ID information correlated and stored beforehand.

In the medical information management system of the present invention, the peculiar biological discrimination pattern is preferable to be at least one of a fingerprint, a palm print, a voiceprint, a face, an iris pattern, a eyeground pattern and a vein pattern.

### Brief Description of Drawings

FIG. 1 is a schematic view showing the structure of a medical information management system 100 according to the present invention;
FIG. 2 is a schematic view showing the structure of a medical information management system 200 according to the present invention;
FIG. 3 is a block diagram showing the structure of principal portions of a management server 1a in a first embodiment of the present invention;
FIG. 4 is a flow chart showing an information store process 1 performed by the control section 11 shown in FIG. 3;
FIG. 5 is a flow chart showing an information usage process 1 performed by the control section 11 shown in FIG. 3;
FIG. 6 is a block diagram showing the structure of principal portions of a management server 1b in a second embodiment of the present invention;
FIG. 7 is a flow chart showing an information usage process 2 performed by the control section 11 shown in FIG. 6;
FIG. 8 is a block diagram showing the structure of principal portions of a management server 1c in a third embodiment of the present invention;
FIG. 9 is a flow chart showing an information store process 2 performed by the control section 11 shown in FIG. 8;
FIG. 10 is a flow chart showing an information usage process 3 performed by the control section 11 shown in FIG. 8;
FIG. 11 is a block diagram showing the structure of principal portions of a management server 1d in a fourth embodiment of the present invention;
FIG. 12 is a flow chart showing an information usage process 4 performed by the control section 11 shown in FIG. 11;
FIG. 13 is a block diagram showing the structure of principal portions of a management server 1e in a fifth embodiment of the present invention;
FIG. 14 is a flow chart showing an information usage process 5 performed by the control section 11 shown in FIG. 13;
FIG. 15 is a block diagram showing the structure of principal portions of a management server 1f in a sixth embodiment of the present invention;
FIG. 16 is a flow chart showing an information usage process 6 performed by the control section 11 shown in FIG. 15;
FIG. 17 is a block diagram showing the structure of principal portions of a management server 1g in a seventh embodiment of the present invention;
FIG. 18 is a flow chart showing an information usage process 7 performed by the control section 11 shown in FIG. 17; and
FIG. 19 is a view showing an example of data structure in a database 16 in a management server 1.

### Best Mode for Carrying Out of the Invention

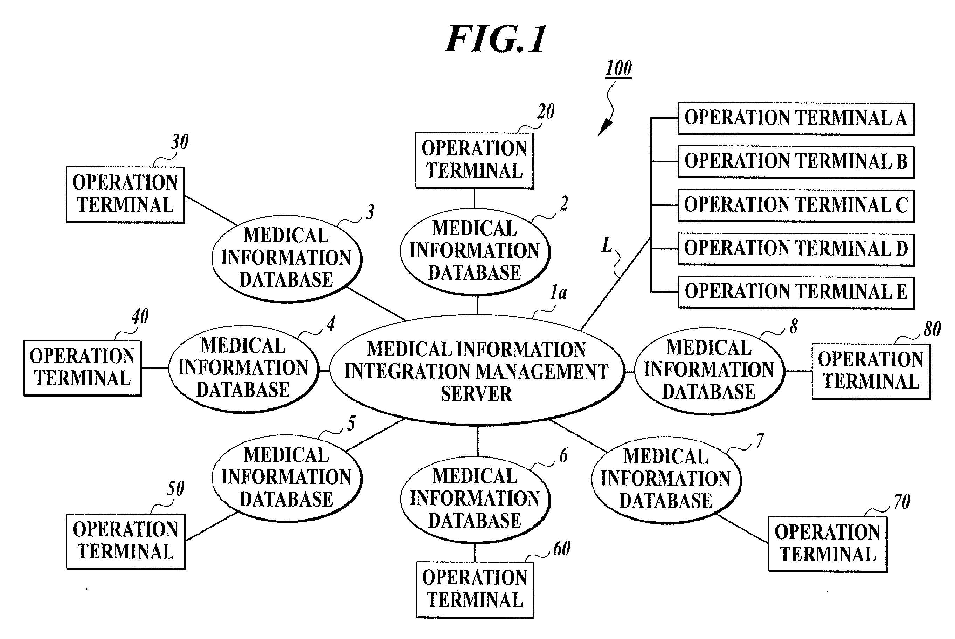

Hereinafter, the embodiments of the present invention will be described in detail with reference to FIGS. 1 to 19. However, the present invention is not to be construed as limited to the examples shown in FIGS. 1 to 19.

The correspondence between the components of a medical information management system according to the present invention and the components of a medical information management system 100 in first to seventh embodiments will now be described clearly. That is, a management device according to the present invention corresponds to medical information integration management servers 1a to 1g in the embodiments, database devices according to the present invention corresponds to medical information databases 2 to 8 in the embodiments, an operation terminal according to the present invention corresponds to operation terminals A to E, 20, 30, 40, 50, 60, 70, and 80 in the embodiments, and an output device according to the present invention corresponds to output devices X to Z in the embodiments.

Moreover, the correspondence between the components of the management device according to the present invention and the components of the medical information integration management servers 1a to 1g in the embodiments will be described clearly. A reception section according to the present invention corresponds to a reception section 15 in the embodiments, a storage section according to the present invention corresponds to a database 16 in the embodiments, an individual verification section according to the present invention corresponds to an individual verification section 12 in the embodiments, a data access authority authentication section according to the present invention corresponds to a data access authority authentication section 13 in the embodiments, a control section and a recording control section according to the present invention correspond to a control section 11 in the embodiments, an output permission section according to the present invention corresponds to an output permission section 14 in the embodiments, a data falsification prevention section according to the present invention corresponds to a data falsification prevention section 17 in the embodiments, and a date and time providing section according to the present invention corresponds to a data and time providing section 18 in the embodiments. Moreover, an automatic transmission section in the database device according to the present invention corresponds to automatic transmission sections 21, 31, 41, 51 and 61 in the respective medical information databases 2 to 6 in the embodiments.

The structure of the embodiments will be described first.

FIG. 1 is a schematic view showing the structure of the medical information management system 100 in which medical information in, for example, one medical institution is consolidated by a medical information integration management server 1a. As shown in FIG. 1, the medical information management system 100 comprises the medical information integration management server (hereinafter referred to as the management server) 1a, the operation terminals A to E for operating the management server 1a, the seven medical information databases (DBs) 2 to 8, and the operation terminals 20, 30, 40, 50, 60, 70, and 80 for operating each of the medical information DBs 2 to 8. Here, medical information stored in the medical information DBs 2 to 8 can be browsed, added, modified, or processed by the use of the operation terminals 20, 30, 40, 50, 60, 70, and 80 respectively.

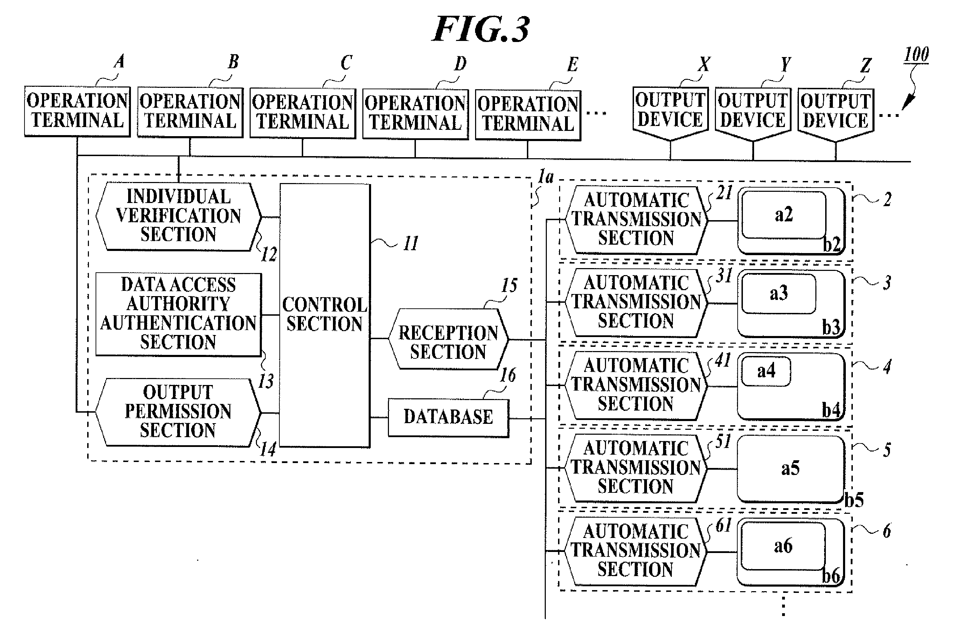

The management server 1a is used for consolidating management of managing medical information. Medical information stored in the database 16 in the management server 1a (see FIG. 3) can be browsed, added, modified, or processed by the use of the operation terminals A to E connected to the management server 1a via a network L. The medical information DBs 2 to 8 are connected to the management server 1a via the network L. Medical information stored in the medical information DBs 2 to 8 can be browsed, added, modified, or processed by the use of the operation terminals 20, 30, 40, 50, 60, 70, and 80 respectively. Moreover, by the use of any of the operation terminals 20, 30, 40, 50, 60, 70, and 80 connected to the medical information DBs 2 to 8 respectively, the management server 1a can be accessed and medical information can be browsed, added, modified, or processed.

Here, the network L connects the server and the plurality of operation terminals and is a network in a limited area used for sharing information or a resource (for example, a database, an output device or the like) among the plurality of operation terminals. By constructing the network L by using a technique, such as the World Wide Web (WWW), TCP/IP or the like, which has been widely applied on the Internet, a network with high reliability can be built speedily and an affinity for the Internet can be improved. Preferably, only specific users can gain access to the network L from the viewpoint of reliability relating to information management.

In FIG. 1, the seven medical information DBs 2 to 8 are shown as an example. However, the number of medical information DBs must be not less than two. There is no other limitation on the number of medical information DBs. In FIG. 1, only one operation terminal 20, 30, 40, 50, 60, 70 or 80 is connected to each of the medical information DBs 2 to 8. However, not less than two or a plurality of operation terminals may be connected to each of the medical information DBs 2 to 8. In this example, the five operation terminals A to E are connected to the management server 1a. However, the operation terminals 20, 30, 40, 50, 60, 70, and 80, which are connected to the medical information DBs 2 to 8 respectively, may be used instead to operate the management server 1a. Alternatively, one or more terminals directly connected to the management server 1a by, for example, a bus may be used.

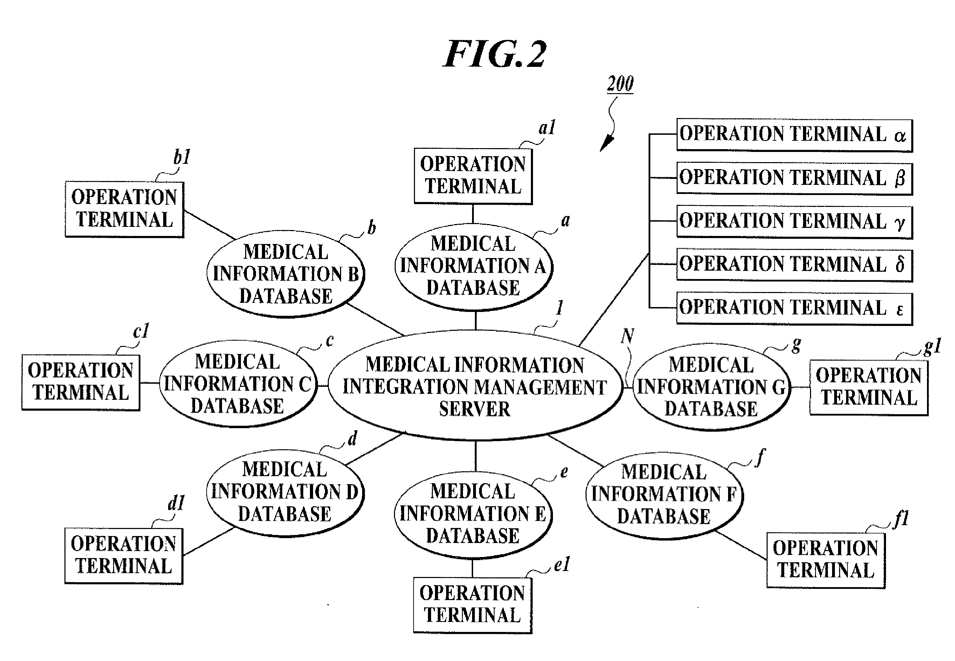

FIG. 2 is a view showing an example of the structure of a medical information management system 200. Similarly to FIG. 1, the medical information management system 200 manages medical information in block. The medical information management system 200 is used for regional medical informatization. Databases operated by medical institutions A to G are connected by a network N and are managed in block by a medical information integration management server (hereinafter referred to as the management server) 1. As shown in FIG. 2, the medical information management system 200 comprises seven medical institution databases a to g operated by the medical institutions A to G respectively and operation terminals a1, b1, c1, d1, e1, f1, and g1 for operating the medical institution DBs a to g respectively. Medical information stored in the medical institution DBs a to g can be browsed, added, modified, or processed by the use of the operation terminals a1, b1, c1, d1, e1, f1, and g1 respectively. For example, the medical information management system 200 may include a plurality of operation terminals a1, a2, a3, ..., and aN by which the medical institution DB a can be manipulated.

The management server 1 is used for consolidating management of managing medical information in the medical information management system 200. Medical information can be browsed, added, modified, or processed by the use of operation terminals α to ε connected to the management server 1. The medical institution DBs a to g are connected to the management server 1 via the network N. By operating one of the operation terminals a1, b1, c1, d1, e1, f1, and g1 and gaining access to the management server 1, medical information can be browsed, added, modified, or processed.

Here, the network N includes various communication lines, such as a telephone line network, an ISDN line network, a dedicated line, a mobile radio communication network, a communication satellite line, a CATV line network and the like, Internet service provider's base stations for connecting them, and the like. The connection of the network N must be able to be established at any time. However, there is no need to establish the connection of the network N at all times. Preferably, only specific users can gain access to the network N from the viewpoint of reliability relating to information management. In particular, it is preferable that the connection between the management server 1 and the operation terminals α to ε for operating the management server 1 should be built with a dedicated line. The reason for this is that the operation terminals α to ε can directly manipulate medical information stored in the management server 1.

Each of the operation terminals a1, b1, c1, d1, e1, f1, and g1 may be the one which gains access to the management server 1 to add, modify, or browse medical information. In FIG. 2, the case that the seven medical institution DBs a to g are provided is described as an example. The number of medical institution DBs must be not less than two. There is no other limitation on the number of medical institution DBs. Further, the case that the five operation terminals α to ε connected to the management server 1 by the network N are provided is described as an example. However, the operation terminals a1, b1, c1, d1, e1, f1, and g1, which are connected to the medical institution DBs a to g respectively, may be used instead to operate the management server 1. Alternatively, one or more terminals directly connected to the management server 1 may be used.

The structure of the two medical information management systems has been described in the above. However, the above-mentioned medical information management systems 100 and 200 are examples, and the systems may comprise various other modes. The structure of each section in the medical information management system 100 is almost the same as that of each section in the medical information management system 200. Moreover, the operation of each section in the medical information management system 100 is almost the same as that of each section in the medical information management system 200. Therefore, first to seventh embodiments will now be described by using a case where the present invention is applied to the medical information management system 100 as an example.

### [First Embodiment]

A management server 1a in the medical information management system 100 will be described first. FIG. 3 is a block diagram showing the structure of principal portions of the management server 1a in the medical information management system 100. In FIG. 3, only components necessary for describing the first embodiment will be shown and the other components and devices will be omitted.

As shown in FIG. 3, the management server 1a comprises a control section 11, an individual verification section 12, a data access authority authentication section 13, an output permission section 14, a reception section 15, a database 16, and the like. These sections are connected by a bus. Moreover, operation terminals A to E for operating the management server 1a and output devices X to Z for outputting medical information in the management server 1a as a hardcopy, which are connected to the management server 1a via a network L, are provided.

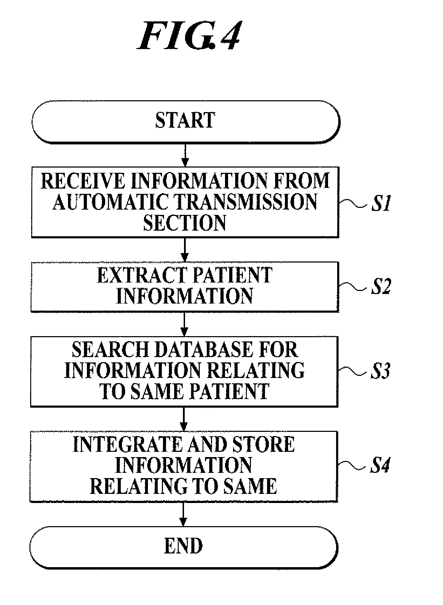

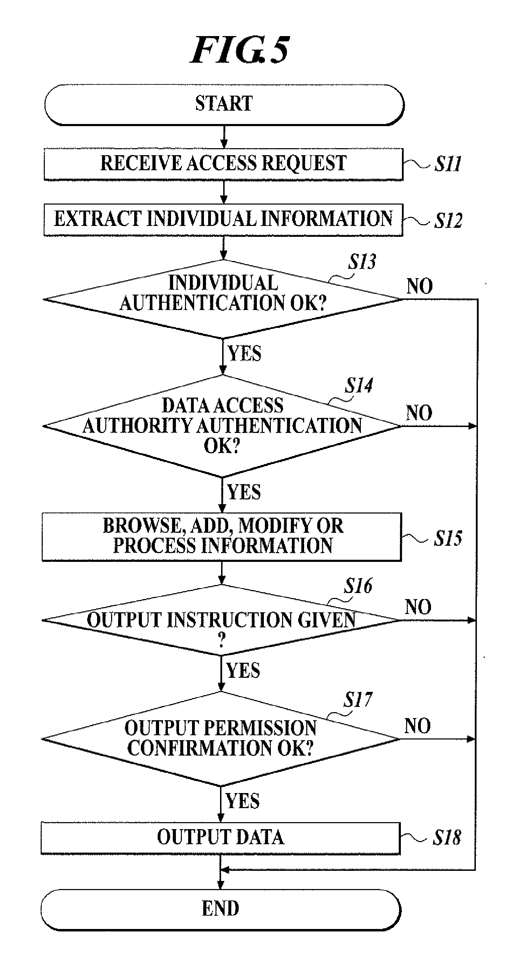

The control section 11 reads and executes various system programs stored in a storage device (not shown) in the database 16 and drives and controls each section in the management server 1a. Concretely, the control section 11 performs an information store process 1 (see FIG. 4) and information usage process 1 (see FIG. 5) described later. When the control section 11 performs the information store process 1, the control section 11 receives medical information sent from one of medical information DBs 2 to 6, extracts patient information from the medical information, and searches the database 16 for medical information relating to the same patient. Then, when there is medical information relating to the same patient in the database 16, then the control section 11 correlates the received medical information with the patient information, integrates this medical information with the medical information which has already stored, and stores the integrated medical information in the database 16.

When the control section 11 performs the information usage process 1, the control section 11 receives an access request sent from one of the operation terminals A to E, extracts personal information relating to an operator of the operation terminals A to E included in the access request, and makes the individual verification section 12 perform personal authentication. If the personal authentication succeeds, then the control section 11 makes the data access authority authentication section 13 verify whether specified medical information can be used. If the specified medical information can be used, then the control section 11 makes the operation terminal operator browse, add, modify, or process the medical information. Moreover, when instructions to output the medical information are inputted from the operation terminal, the control section 11 makes the output permission section 14 verify whether the specified medical information can be outputted. When the medical information can be outputted, the control section 11 makes the output devices X to Z output it through the output permission section 14.

The individual verification section 12 receives an access request sent from one of the operation terminals A to E via the network L. The individual verification section 12 obtains personal information relating to an operator who is operating the operation terminal A to E from the access request and judges from this personal information whether or not the operator who is operating the operation terminal A to E has access authority. In this case, the personal information is used for specifying a person and includes a password, ID card information, a fingerprint, a palm print, a voiceprint, a face (its outline, for example), a signature handwriting, an iris pattern, an eyeground pattern, or a vein (blood vessel) pattern (in a palm, a finger, an arm, or the like). Personal authentication will be performed by comparing this personal information with information relating to an operator having access authority which has been registered in advance in the database 16.

It is preferable that at least one of a password, an ID card, a fingerprint, a palm print, a voiceprint, a face (its outline, for example), a signature handwriting, an iris pattern, an eyeground pattern, and a vein (blood vessel) pattern is used as personal information (biological discrimination pattern). Information, such as a fingerprint, a palm print, a voiceprint, a face, an iris pattern, an eyeground pattern, or a vein pattern, specific to each person is more preferable to be used from the viewpoint of security. Moreover, in order to increase the security further, a combination of one of a password and an ID card and at least one of a fingerprint, a palm print, a voiceprint, a face, an iris pattern, an eyeground pattern, and a vein pattern may be used as personal information.

As a result, personal authentication of an operator can be performed reliably on the basis of various pieces of information and the security of the medical information management system can be improved. In addition, by combining a plurality of pieces of personal information, unauthorized access to the system by a third person can be prevented reliably. In addition, making it possible to apply various pieces of personal information will lead to an increase in the versatility of the system.

Operators having access authority include doctors, radiologists, nurses, pharmacists, clerical employees such as accountants and the like, dietitians, nursing care helpers who are engaged in home-visit nursing care or the like, patients and the like. These operators can be registered arbitrarily from among persons who need medical information stored in the management server 1a. The individual verification section 12 may be included in each of the operation terminals A to E. As a result, personal authentication will be performed by the individual verification section 12 in each of the operation terminals A to E before the operation terminals A to E are connected to the management server 1a. Moreover, the data access authority authentication section may include the reception section. In this case, an access request will directly be sent from each of the operation terminals A to E to the data access authority authentication section according to the instructions given by an operator authenticated by the individual verification section 12 in each of the operation terminals A to E.

The data access authority authentication section 13 identifies medical information, which is stored in the database 16 and on which permission to browse, add, modify, or process is given. In this case, medical information which can be browsed, added, modified, or processed is at least one kind of data selected from clinical examination management system data, radiation department information system data, hospital information system data, electronic chart system data, case management system data, medicine history management system data, medicine document data, nursing-care insurance system data, and medical-related document data. Preferably, medical information which can be browsed, added, modified, or processed is two or more kinds of data selected from the above-mentioned data.

The data access authority authentication section 13 may identify accessible medical information on the basis of personal information relating to an operator, obtained by the individual verification section 12. For example, when only doctors, radiologists, nurses, pharmacists, and clerical employees such as accountants and the like, in a hospital are operators, it is preferable that accessible medical information should be two or more kinds of data selected from clinical examination management system data, radiation department information system data, hospital information system data, electronic chart system data, case management system data, and medicine history management system data.

Preferably, the kind of data which can be browsed, added, modified, or processed by an operator authenticated by the individual verification section 12 is at least one kind of data selected from character data, on-off data, static image data, and dynamic image data. Further, when data which can be added, modified, or processed is character data or on-off data, an input member (not shown) included in each of the operation terminals A to E is at least one kind of input member selected from a pen, a keyboard, a mouse, and voice. This selection can be carried out properly according to how a person who operates each of the operation terminals A to E is experienced. On-off data is data indicated by on/off, and means alternative information, such as sex, the presence of a clinical history or the like.

The output permission section 14 judges from the personal information obtained by the individual verification section 12 and the result of a judgment made by the data access authority authentication section 13 whether permission to record medical information stored in the management server 1a in another record medium as electronic information or to output medical information stored in the management server 1a as a hardcopy should be given.

The reception section 15 receives medical information sent from automatic transmission sections 21, 31, 41, 51, and 61 included in the medical information DBs 2 to 6 respectively. The medical information sent from the automatic transmission sections 21, 31, 41, 51, and 61 will be described in detail later.

The database 16 includes a record medium (not shown) where programs, data and the like are stored in advance. This record medium is a magnetic or optical record medium or a semiconductor memory. This record medium is mounted on the database 16 so that it cannot be removed or so that it can be removed freely. This record medium stores a system program, various processing programs corresponding to the system, data processed by the various processing programs, and the like. These programs will be stored in the form of computer readable program codes and the control section 11 will sequentially perform operation in accordance with the program codes.

Part or all of programs, data and the like to be stored in this record medium may be received from another device, such as a server, a client or the like, by the reception section 15 via a network, such as a WAN, LAN or the like. In addition, a record medium in a server on a network may be used instead. Moreover, the programs may be transferred to a server or a client via a transmission medium, such as a network or the like, and be installed there.

Concretely, the database 16 stores medical information sent from the medical information DBs 2 to 6. This medical information includes, for example, clinical examination management system data, radiation department information system data, hospital information system data, electronic chart system data, case management system data, medicine history management system data, medicine document data, nursing-care insurance system data, and medical-related document data. This medical information is integrated according to patients on the basis of patient information included in medical information sent from the medical information DBs 2 to 6 and is managed and stored. This patient information is used for specifying a patient and includes a fingerprint, a voiceprint, a face (its outline, for example), a signature handwriting, an iris pattern, an eyeground pattern, a vein (blood vessel) pattern, and dental treatment information besides information, such as a name, an age, and an address, recorded on a health insurance card. When adding, modifying or processing is performed on medical information stored in the database 16, the operation result will be stored.

The database 16 also stores personal information relating to persons who have been registered as operators having the authority to gain access to the management server 1a. This personal information is used for specifying a person and includes a password, ID card information, a fingerprint, a voiceprint, a face (its outline, for example), a signature handwriting, an iris pattern, an eyeground pattern, or a vein (blood vessel) pattern. This personal information is used by the individual verification section 12 for authenticating an operator of each of the operation terminals A to E who sent an access request to the management server 1a.

The operation terminals A to E are connected to the management server 1a via the network L. Each of the operation terminals A to E sends an access request including operating instructions to browse, add, modify, or process medical information stored in the management server 1a to the management server 1a. This access request includes personal information for authenticating an operator. Whether the operator is a person with access authority will be verified on the basis of this personal information. If authentication succeeds, then the operating instructions will be accepted. Moreover, each of the operation terminals A to E sends instructions for outputting medical information from any of the output devices X to Z to the management server 1a.

The output devices X to Z are connected to the management server 1a and operation terminals A to E via the network L. Each of the output devices X to Z may output medical information to a record medium, such as CD-R, CD-RW, DVD-R, DVD-RW, a hard disc, a Blu-ray disc, a large-capacity hologram used in the apparatus disclosed in Japanese Patent Laid-Open Publication No. 2002-83431, Japanese Patent Laid-Open Publication No. 2002-123948, Japanese Patent Laid-Open Publication No. 2002-123949, Japanese Patent Laid-Open Publication No. 2002-183975, or the like, a thermal silver salt record medium, a photothermal silver salt record medium, a thermal diffusive dye transfer record medium, an ink-jet record medium, a record medium used in electrophotography, or the like as visualized information or a hardcopy.

Next, the medical information DBs 2 to 6 will be described.

The medical information DBs 2 to 6 (hereinafter, it is integrally described as "medical information DBs 2") is operated by operation terminals 20, 30, 40, 50, and 60 (hereinafter, it is integrally described as "operation terminals 20") respectively, and comprises automatic transmission sections 21, 31, 41, 51, and 61 (hereinafter, it is integrally described as "automatic transmission sections 21"), respectively, for automatically sending necessary information among medical information inputted via an input section (not shown) of the operation terminals 20 to the management server 1a.

Concretely, the medical information sent to the management server 1a by the automatic transmission sections 21 is at least one kind of data selected from clinical examination management system data, radiation department information system data, hospital information system data, electronic chart system data, case management system data, medicine history management system data, medicine document data, nursing-care insurance system data, and related-related document data. Preferably, this medical information is two or more kinds of data selected from the above-mentioned data. Moreover, when only doctors, radiologists, nurses, pharmacists, and clerical employees such as accountants and the like, in a hospital use this medical information, it is preferable that the medical information should be two or more kinds of data selected from clinical examination management system data, radiation department information system data, hospital information system data, electronic chart system data, case management system data, and medicine history management system data.

The automatic transmission sections 21 automatically send medical information inputted via the operation terminals 20 and stored in the medical information DBs 2, to the management server 1a to store it. To simplify data processing in the management server 1a, at this time the automatic transmission sections 21 detect the differential between the medical information to be sent and the medical information sent to the management server 1a in the past, and sends only added or modified medical information to the management server 1a. For example, as shown in FIG. 3, the medical information DB 2 stores medical information a2+b2 and only a2 will be sent to the management server 1a via the network as necessary information. Medical information a3, a4, a5, and a6 stored in the medical information DBs 3 to 6, respectively, will also be sent to the management server 1a via the network and be stored there.

When the automatic transmission section 21 sends medical information to the management server 1a to store it in the database 16, the automatic transmission section 21 may add the date and time when the medical information was created to the medical information to be sent as attached information. This enables the control section 11 in the management server 1a to compare the time when the medical information sent this time was created and the date and time when the medical information sent and stored the last date and time was created, and to store only medical information newly added or modified in the database 16. Therefore, information processing can be performed simply.

Time taken for the automatic transmission section 21 to send medical information from the medical information DB 2 to the management server 1a will depend on the structure of the individual database or the system such as a server, backup or the like. It is preferable that medical information should be sent during a period of time, such as between midnight and early morning, when the medical information DBs 2 are not used frequently.

The operation terminals 20 are used for performing browsing, adding, modifying and processing medical information stored in the medical information DBs 2. The operation terminals 20 are connected to the management server 1a via the network L and can be used for browsing, adding, modifying and processing medical information stored in the management server 1a. In this case, the operation terminals 20 send personal information relating to an operator, together with an access request, to the management server 1a. When authentication succeeds, then the access request will be accepted.

Next, operation performed in the first embodiment will be described.

As a premise of the description of the operation, a program for performing each process described on the following flow chart is stored in the database 16 in the form of a computer-readable program code, and the control section 11 sequentially performs operation in accordance with the program code. Moreover, the control section 11 can sequentially perform operation specific to the first embodiment by the use of a program and data supplied from the outside via a transmission medium.

FIG. 4 is a flow chart showing the information store process 1 performed by the control section 11. As shown in FIG. 4, when the control section 11 receives medical information from the medical information DB 2 via the reception section 15 (step S1), the control section 11 extracts patient information from the medical information (step S2) and searches the database 16 for medical information relating to the same patient (step S3). Then, when medical information relating to the same patient has been stored in the database 16, the control section 11 integrates these pieces of medical information, stores them in the database 16 (step S4), and terminates the information store process.

FIG. 5 is a flow chart showing the information usage process 1 performed by the control section 11. As shown in FIG. 5, when the control section 11 receives an access request via the individual verification section 12 (step S11), the control section 11 makes the individual verification section 12 extract personal information and perform personal authentication on an operator who is operating one of the operation terminals A to E which sent the access request (step S12). When personal authentication succeeds (step S13; YES), then the control section 11 makes the data access authority authentication section 13 judge whether medical information access to which was requested is accessible or not (step S14).

When the medical information access to which was requested is accessible (step S14; YES), then the control section 11 makes the operator browse, add, modify or process the medical information stored in the database 16 in accordance with the access request (step S15). Moreover, when instructions to output the medical information are inputted from the operation terminal (step S16; YES), the control section 11 makes the output permission section 14 judge whether the specified medical information can be outputted (step S17). When the medical information can be outputted, then the control section 11 makes the output devices X to Z output the medical information through the output permission section 14 (step S18) and terminates the information usage process.

As described above, the management server 1a stores and manages medical information automatically sent by the automatic transmission sections 21 in the medical information DBs 2 according to patients on the basis of patient information. This enables blanket management of medical information in the medical information management system 100 and prevents the same medical information from being stored repeatedly in the management server 1a. As a result, resources in the medical information management system 100 are used effectively and medical information can be managed efficiently.

To use medical information stored in the management server 1a, personal authentication is performed first by the individual verification section 12 on an operator who is operating one of the operation terminals A to E. Then, access to the medical information is permitted after medical information to which the operator authenticated by the individual verification section 12 can access is verified by the data access authority authentication section 13. Therefore, a third person cannot access the medical information stored in the management server 1a without permission. This prevents a fraudulent activity, such as intentionally browsing, adding, modifying or processing the medical information stored in the management server 1a, and improves the reliability of the medical information stored in the management server 1a.

Moreover, when instructions to record medical information on a record medium as electronic information or to output medical information as a hardcopy are inputted, whether the specified medical information can be outputted is judged by the output permission section 14. When permission to output the medical information is given, then it will be outputted by the output devices X to Z. This prevents outflow of personal information relating to a patient to the outside and copying of medical information to another record medium. As a result, invasion of privacy can be prevented, and the security of information management can be enhanced.

In FIG. 3, five medical information DBs 2 to 6 for automatically sending information necessary to the management server 1a are described. The number of medical information DBs must be not more than two. There is no other limitation on the number of medical information DBs. Moreover, in FIG. 3, five operation terminals A to E for operating the management server 1a are provided. However, a plurality of operation terminals may be located at need. The operation terminals A to E may be located at different places via the network L and the Internet according to the uses. As described in FIG. 1, any of the operation terminals 20, 30, 40, 50, 60, 70, and 80 provided to the medical information DBs 2 to 8, respectively, may be used for accessing the management server 1a and adding, modifying or browsing medical information stored therein. Similarly, as described in FIG. 2, any of the operation terminals a1, b1, c1, d1, e1, f1, and g1 provided to the medical institution DBs a to g, respectively, may be used for accessing the management server 1 and adding, modifying or browsing medical information stored therein. In addition, in FIG. 3, the three output devices X to Z are described. The number of output devices must be at least one. There is no other limitation on the number of output devices. Furthermore, these output devices may be located at different places via the network L. The output devices X to Z may be connected directly to the operation terminals A to E. The same is applied to the second to seventh embodiments described later.

As stated above, the management server 1a integrates and manages medical information sent by the automatic transmission section 21, 31, 41, 51, and 61 in the medical information DBs 2 to 6, respectively, according to patients on the basis of patient information. To use the medical information stored in the management server 1a, personal authentication is performed first by the individual verification section 12 on an operator who is operating one of the operation terminals A to E. Then, access to the medical information is permitted after medical information to which the operator authenticated by the individual verification section 12 can access is verified by the data access authority authentication section 13.

### [Second Embodiment]

Next, the second embodiment of the present invention will be described.

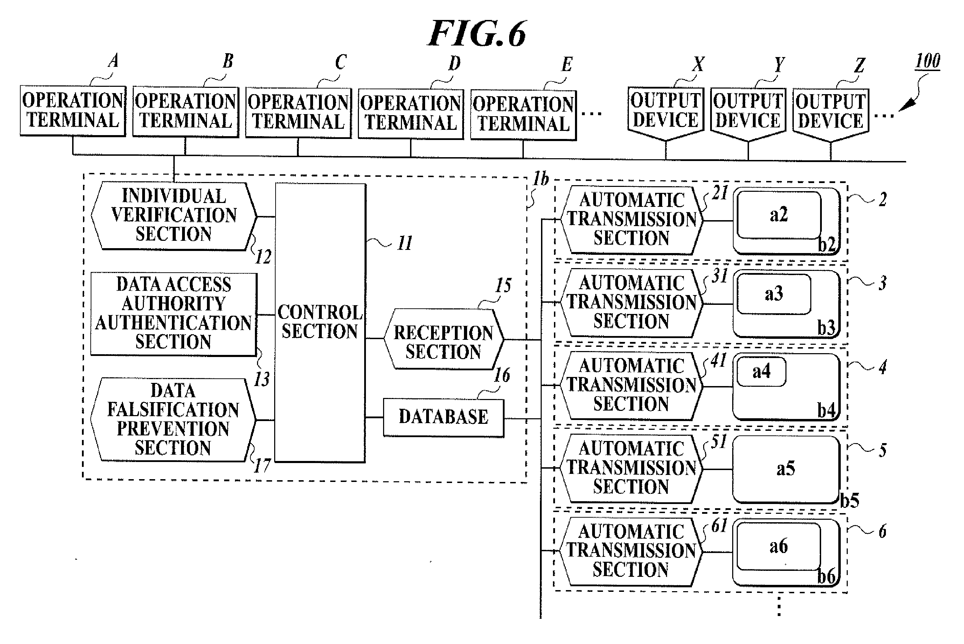

FIG. 6 is a block diagram showing the structure of principal portions of a management server 1b in the second embodiment. As shown in FIG. 6, the management server 1b comprises a control section 11, an individual verification section 12, a data access authority authentication section 13, a reception section 15, a database 16, a data falsification prevention section 17, and the like. That is, compared with the management server 1a in the above-mentioned first embodiment, the management server 1b newly comprises the data falsification prevention section 17 in place of the output permission section 14. The structure except the data falsification prevention section 17 of the management server 1b is the same as that of the management server 1a in the above first embodiment, so detailed descriptions of the same structure will be omitted. Hereinafter, structure and operation characteristic of the second embodiment will be described.

When adding, modifying or processing is performed to the medical information stored in the database 16, the data falsification prevention section 17 prevents the medical information from being falsified by holding the original information and storing a change in the original information as a history. This not only prevents important medical information from being rewritten accidentally but prevents a fraudulent activity, such as intentionally adding, modifying or processing the medical information stored in the database 16. Therefore, the security of the stored medical information can be ensured.

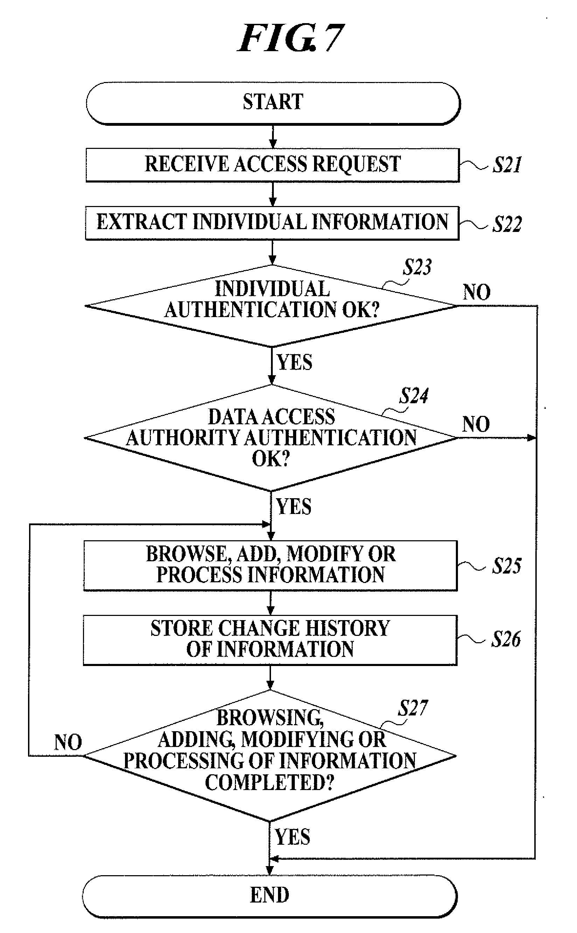

Next, operation performed in the second embodiment will be described. The control section 11 in the management server 1b performs an information usage process 2 as a process characteristic of the second embodiment. FIG. 7 is a flow chart showing the information usage process 2 performed by the control section 11. As shown in FIG. 7, when the control section 11 receives an access request via the individual verification section 12 (step S21), the control section 11 makes the individual verification section 12 extract personal information and perform personal authentication on an operator who is operating one of the operation terminals A to E which sent the access request (step S22). When personal authentication is succeeded (step S23; YES), then the control section 11 makes the data access authority authentication section 13 judge whether the medical information access to which was requested is accessible or not (step S24).

When the medical information access to which was requested is accessible (step S24; YES), then the control section 11 makes the operator browse, add, modify or process the medical information stored in the database 16 in accordance with the access request (step S25). When the medical information stored in the database 16 is added, modified or processed, the control section 11 makes the data falsification prevention section 17 correlate a history indicative of this change with the medical information and store it (step S26). The control section 11 judges whether the adding, modifying or processing of the medical information is completed (step S27). When the changing of the medical information is completed (step S27; YES), then the control section 11 terminates the information usage process 2. When the change of the medical information is not completed (step S27; NO), then the control section 11 returns to step S25 to repeat the above process.

As described above, in the second embodiment, when medical information stored in the database 16 in the management server 1b is added, modified or processed, the management server 1b makes the data falsification prevention section 17 hold the original medical information and store a history indicative of this change by correlating it with the medical information. This prevents the medical information stored in the database 16 from being rewritten or falsified illegally. As a result, the reliability of medical information can be improved. Furthermore, the original medical information is held and a change history is stored. Accordingly, even when medical malpractice or the like has occurred, suppression of information by erasing a disadvantageous record will be impossible.

### [Third Embodiment]

Next, the third embodiment of the present invention will be described.

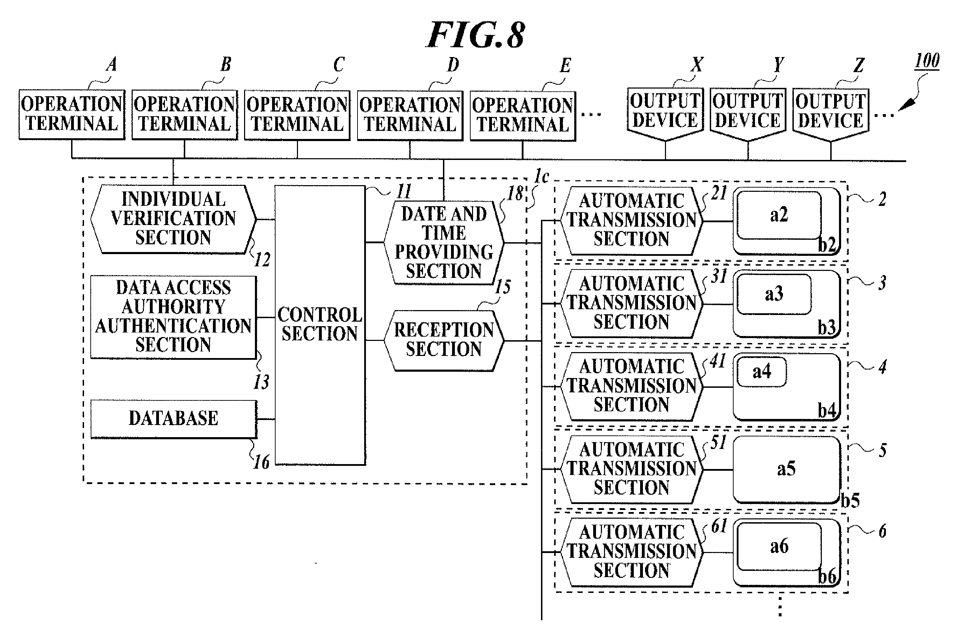

FIG. 8 is a block diagram showing the structure of principal portions of a management server 1c in the third embodiment. As shown in FIG. 8, the management server 1c comprises a control section 11, an individual verification section 12, a data access authority authentication section 13, a reception section 15, a database 16, a date and time providing section 18, and the like. That is, compared with the management server 1a in the above first embodiment, the management server 1c newly comprises the date and time providing section 18 in place of the output permission section 14. The structure except the date and time providing section 18 of the management server 1c is the same as that of the management server 1a in the above first embodiment, so detailed descriptions of the same structure will be omitted. Hereinafter, structure and operation characteristic of the third embodiment will be described.

The date and time providing section 18 adds the date and time when medical information sent from the medical information DBs 2 to 6 was received by the reception section 15 as attached information. The date and time providing section 18 also adds the date and time when medical information stored in the database 16 in the management server 1c was browsed, added, modified or processed as attached information. The date and time providing section 18 may add the date and time when information received by the reception section 15 was stored in the database 16 as attached information.

As a result, when new medical information was sent to or stored in the management server 1c or when and how medical information stored in the database 16 in the management server 1c was browsed, added, modified or processed can be checked ex post facto, and how the stored medical information has changed can be grasped easily. Therefore, when medical information is clinical examination management system data, radiation department information system data, hospital information system data, electronic chart system data, case management system data, medicine history management system data, and the like, a change in the state of a patient can be observed on a time-series basis. This will improve the accuracy and efficiency of a doctor's diagnosis.

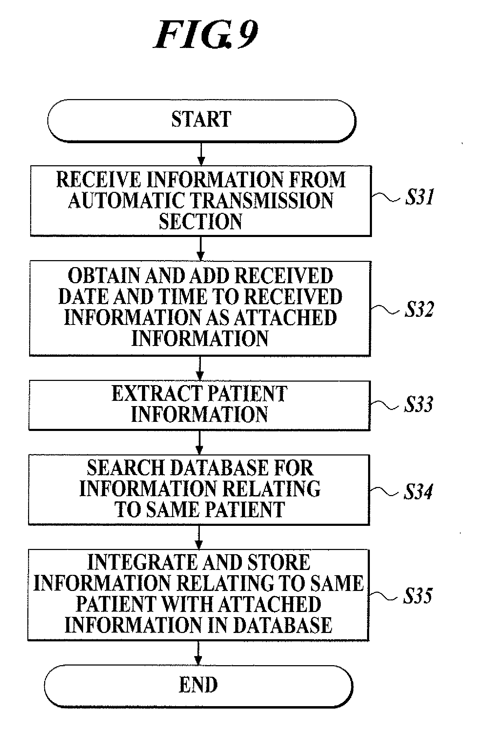

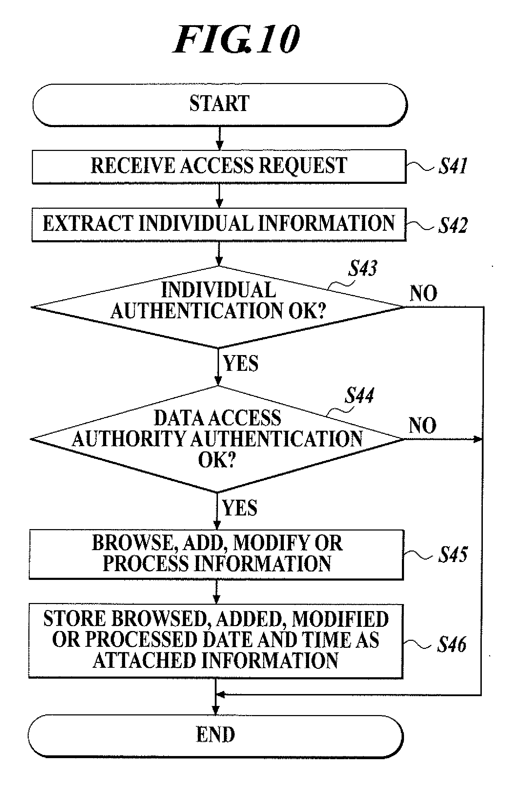

Next, operation performed in the third embodiment will be described. The control section 11 performs the later-described information store process 2 (see FIG. 9) and information usage process 3 (see FIG. 10) as processes characteristic of the third embodiment.

FIG. 9 is a flow chart showing the information store process 2 performed by the control section 11. As shown in FIG. 9, when the control section 11 receives medical information from the medical information DB 2 via the reception section 15 (step S31), the control section 11 makes the date and time providing section 18 obtain the date and time when the control section 11 received the medical information and add it to the received medical information as attached information (step S32).

Then the control section 11 extracts patient information from the received medical information (step S33), and searches the database 16 for medical information relating to the same patient (step S34). When the medical information relating to the same patient has been stored in the database 16, the control section 11 integrates these pieces of medical information, stores them in the database 16 with the attached information (step S35), and terminates the information store process 2.

FIG. 10 is a flow chart showing the information usage process 3 performed by the control section 11. As shown in FIG. 10, when the control section 11 receives an access request via the individual verification section 12 (step S41), the control section 11 makes the individual verification section 12 extract personal information (step S42) and perform personal authentication on an operator who is operating one of the operation terminals A to E which sent the access request. When personal authentication is succeeded (YES in step S43), then the control section 11 makes the data access authority authentication section 13 judge whether the medical information access to which was requested is accessible or not (step S44).

When the medical information access to which was requested is accessible (step S44; YES), then the control section 11 makes the operator browse, add, modify or process the medical information stored in the database 16 in accordance with the access request (step S45). Then, the control section 11 makes the date and time providing section 18 obtain the date and time when the medical information was browsed, added, modified or processed, correlates it with the medical information as attached information, stores it in the database 16 (step S46), and terminates the information usage process 3.

As described above, in the third embodiment, the management server 1c adds the date and time when new medical information was stored in the database 16 or the date and time when the medical information stored in the database 16 was added, modified or processed to the medical information by the date and time providing section 18. As a result, a change in medical information can be checked on a time-series basis and a change in the state of a patient can be recognized accurately. This will improve the accuracy and efficiency of a doctor's diagnosis. Moreover, by specifying the date and time when medical information was added, modified or processed, the act of illegally operating medical information will be restrained.

### [ Fourth Embodiment]

Next, the fourth embodiment of the present invention will be described.

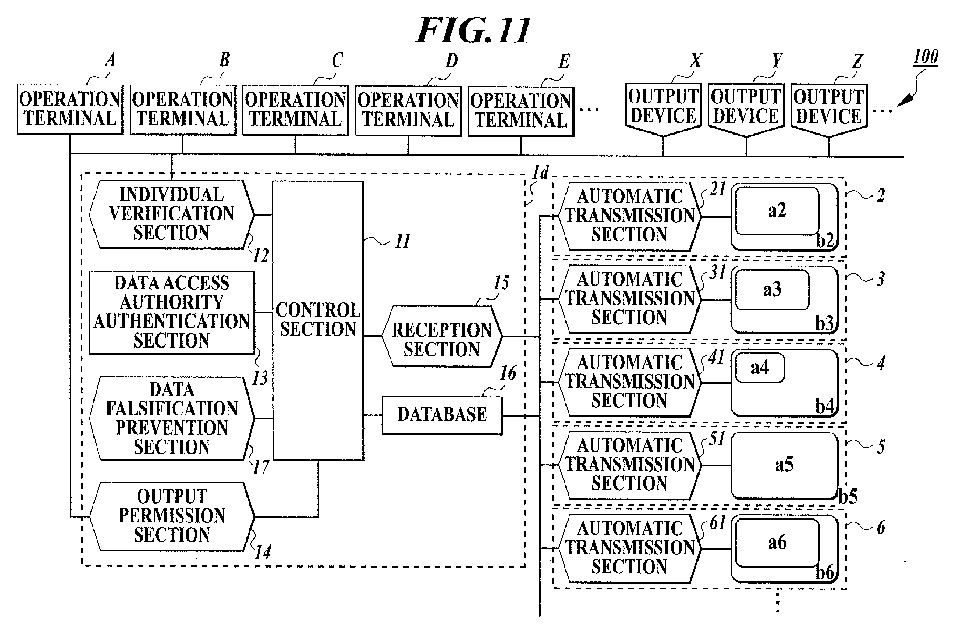

FIG. 11 is a block diagram showing the structure of principal portions of a management server 1d in the fourth embodiment. As shown in FIG. 11, the management server 1d comprises a control section 11, an individual verification section 12, a data access authority authentication section 13, an output permission section 14, a reception section 15, a database 16, a data falsification prevention section 17, and the like. That is, compared with the management server 1a in the above first embodiment, the management server 1d further comprises the data falsification prevention section 17. The structure of the management server 1d are the same as that of the management server 1a in the above first embodiment or the management server 1b in the above second embodiment, so that detailed descriptions of them will be omitted. Hereinafter, operation characteristic of the fourth embodiment will be described.

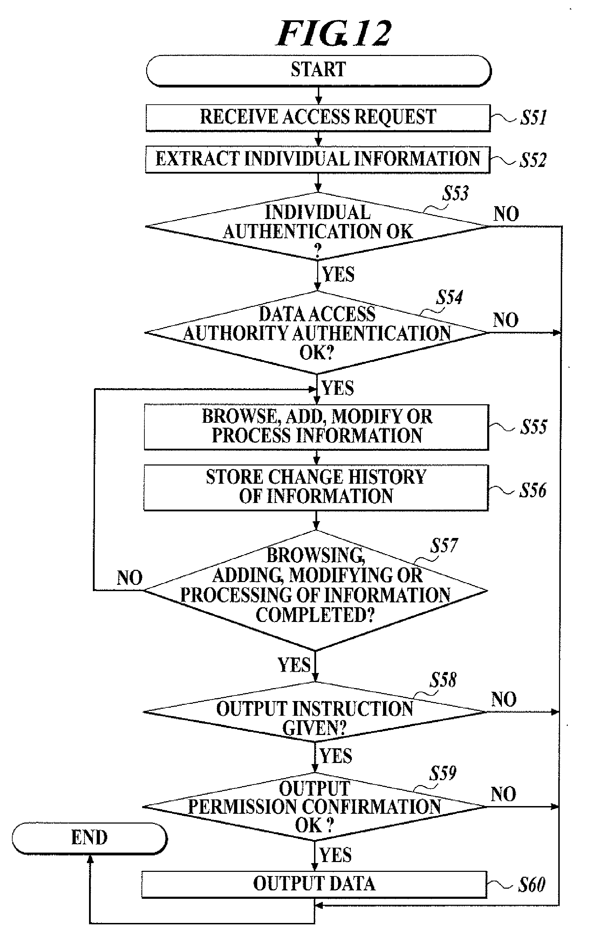

The control section 11 performs an information usage process 4 (see FIG. 12) described later as a process characteristic of the fourth embodiment. FIG. 12 is a flow chart showing the information usage process 4 performed by the control section 11. As shown in FIG. 12, when the control section 11 receives an access request via the individual verification section 12 (step S51), the control section 11 makes the individual verification section 12 extract personal information and perform personal authentication on an operator who is operating one of the operation terminals A to E which sent the access request (step S52). When personal authentication is succeeded (step S53; YES), then the control section 11 makes the data access authority authentication section 13 judge whether medical information access to which was requested is accessible or not (step S54).

When the medical information access to which was requested is accessible (step S54; YES), then the control section 11 makes the operator browse, add, modify or process the medical information stored in the database 16 in accordance with the access request (step S55). When the medical information stored in the database 16 is added, modified or processed, the control section 11 makes the data falsification prevention section 17 correlate a history indicative of this change with the medical information and store it (step S56). The control section 11 judges whether the adding, modifying, or processing of the medical information is completed or not (step S57). When the change of the medical information is completed (step S57; YES), then the control section 11 judges whether instructions to output the medical information have been inputted from the operation terminals A to E (step S58).

When instructions to output the medical information have been inputted from the operation terminals A to E (step S58; YES), then the control section 11 makes the output permission section 14 judge whether the specified medical information can be outputted (step S59). When the medical information can be outputted, then the control section 11 makes the output devices X to Z output the medical information through the output permission section 14 (step S60), and terminates the information usage process 4.

As described above, in the fourth embodiment, when medical information stored in the database 16 in the management server 1d is added, modified or processed, the management server 1d makes the data falsification prevention section 17 hold the original medical information and store a history indicative of this change by correlating it with the medical information. Furthermore, the management server 1d makes the output permission section 14 judge whether the specified medical information can be outputted. When permission to output the medical information is given, then the management server 1d makes the output devices X to Z output it. This prevents a fraudulent activity, such as intentionally browsing, adding, modifying, or processing medical information stored in the management server 1d, outflow of personal information relating to a patient to the outside, and copying of medical information to another record medium. As a result, the security of information management can be enhanced.

### [Fifth Embodiment]

Next, the fifth embodiment of the present invention will be described.

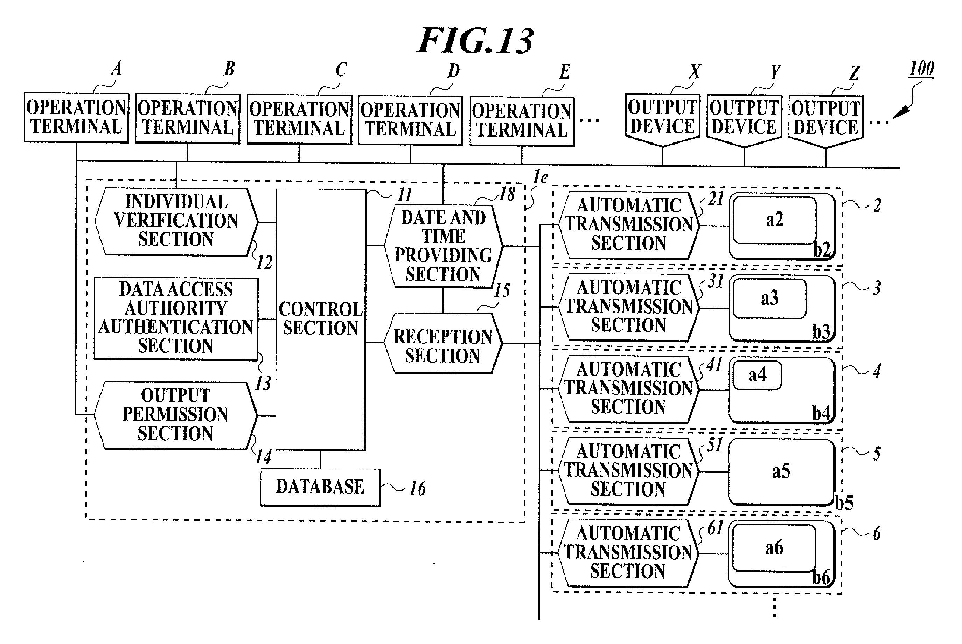

FIG. 13 is a block diagram showing the structure of principal portions of a management server 1e in the fifth embodiment. As shown in FIG. 13, the management server 1e comprises a control section 11, an individual verification section 12, a data access authority authentication section 13, an output permission section 14, a reception section 15, a database 16, a date and time providing section 18, and the like. That is, compared with the management server 1a in the above first embodiment, the management server 1e further includes the date and time providing section 18. The structure of the management server 1e are the same as that of the management server 1a in the above first embodiment or the management server 1c in the above third embodiment, so that detailed descriptions of them will be omitted. Hereinafter, operation characteristic of the fifth embodiment will be described.

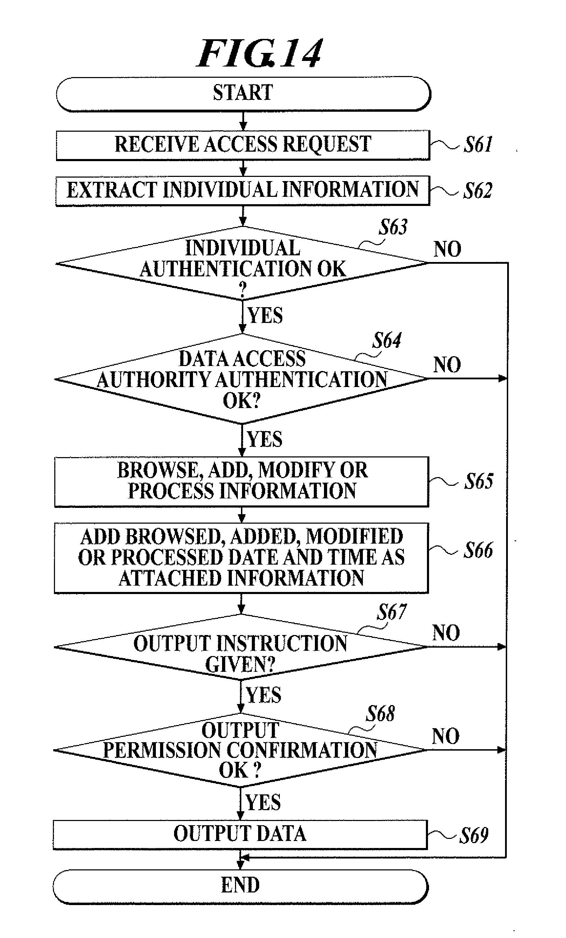

The control section 11 performs an information store process 2 (see FIG. 9) and an information usage process 5 (see FIG. 14) described later as processes characteristic of the fifth embodiment. The information store process 2 is the same as that in the above third embodiment, so that descriptions of it will be omitted.

FIG. 14 is a flow chart showing the information usage process 5 performed by the control section 11. As shown in FIG. 14, when the control section 11 receives an access request via the individual verification section 12 (step S61), the control section 11 makes the individual verification section 12 extract personal information (step S62), and perform personal authentication on an operator who is operating one of the operation terminals A to E which sent the access request. When personal authentication is succeeded (step S63; YES), then the control section 11 makes the data access authority authentication section 13 judge whether the medical information access to which was requested is accessible or not (step S64).

When the medical information access to which was requested is accessible (step S64; YES), then the control section 11 makes the operator browse, add, modify or process the medical information stored in the database 16 in accordance with the access request (step S65). Then, the control section 11 makes the date and time providing section 18 obtain the date and time when the medical information was browsed, added, modified or processed and add it to the medical information as attached information (step S66).

The control section 11 judges whether instructions to output the medical information have been inputted from the operation terminals A to E (step S67). When instructions to output the medical information have been inputted (step S67; YES), then the control section 11 makes the output permission section 14 judge whether the specified medical information can be outputted (step S68). When the medical information can be outputted (step S68; YES), then the control section 11 makes the output devices X to Z output the medical information through the output permission section 14 (step S69), and terminates the information usage process 5.

As described above, in the fifth embodiment, the management server 1e makes the date and time providing section 18 correlate the date and time when new medical information was stored in the database 16 or the date and time when medical information stored in the database 16 was added, modified or processed with the medical information and store it. In addition, the management server 1e makes the output permission section 14 judge whether specified medical information can be outputted. When permission to output the medical information is given, then the management server 1e makes the output devices X to Z output it.

As a result, a change in medical information can be checked on a time-series basis and a change in the state of a patient can be recognized accurately. This will improve the accuracy and efficiency of a doctor's diagnosis. Moreover, by specifying the date and time when medical information was added, modified or processed, the act of subsequently and illegally manipulating medical information stored in the database 16 will be restrained. Furthermore, outflow of personal information relating to a patient to the outside or copying of medical information to another record medium can be prevented. The security of information management therefore can be enhanced.

### [Sixth Embodiment]

Next, the sixth embodiment of the present invention will be described.

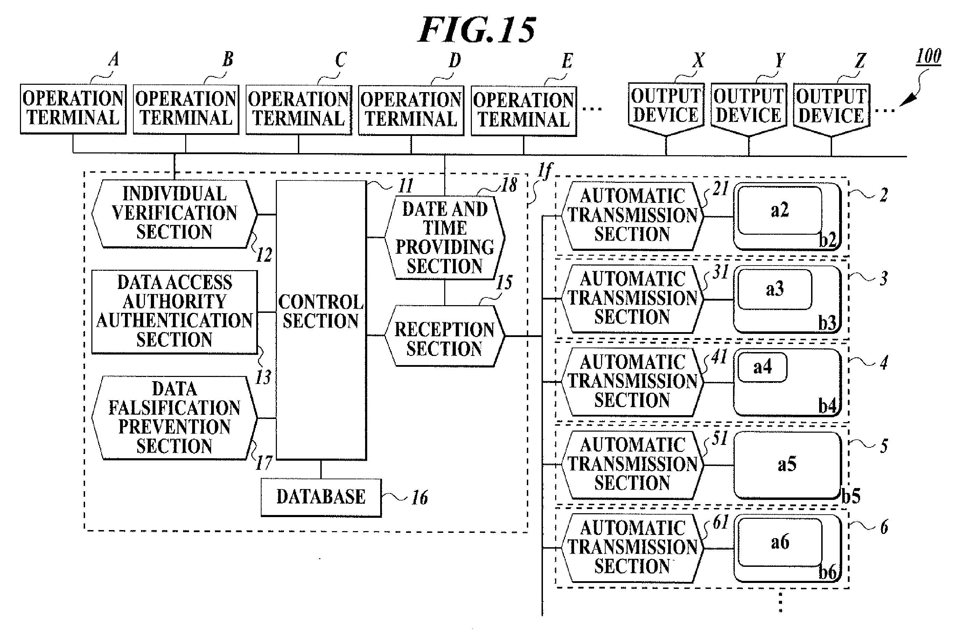

FIG. 15 is a block diagram showing the structure of principal portions of a management server 1f in the sixth embodiment. As shown in FIG. 15, the management server 1f comprises a control section 11, an individual verification section 12, a data access authority authentication section 13, a reception section 15, a database 16, a data falsification prevention section 17, a date and time providing section 18, and the like. That is, compared with the management server 1a in the above first embodiment, the management server 1f comprises the data falsification prevention section 17 and the date and time providing section 18 in place of the output permission section 14. The structure of the management server 1f is the same as that of the management server 1a in the above first embodiment, the management server 1b in the above second embodiment, or the management server 1c in the above third embodiment, so that detailed descriptions of the same structure will be omitted. Hereinafter, operation characteristic of the sixth embodiment will be described.

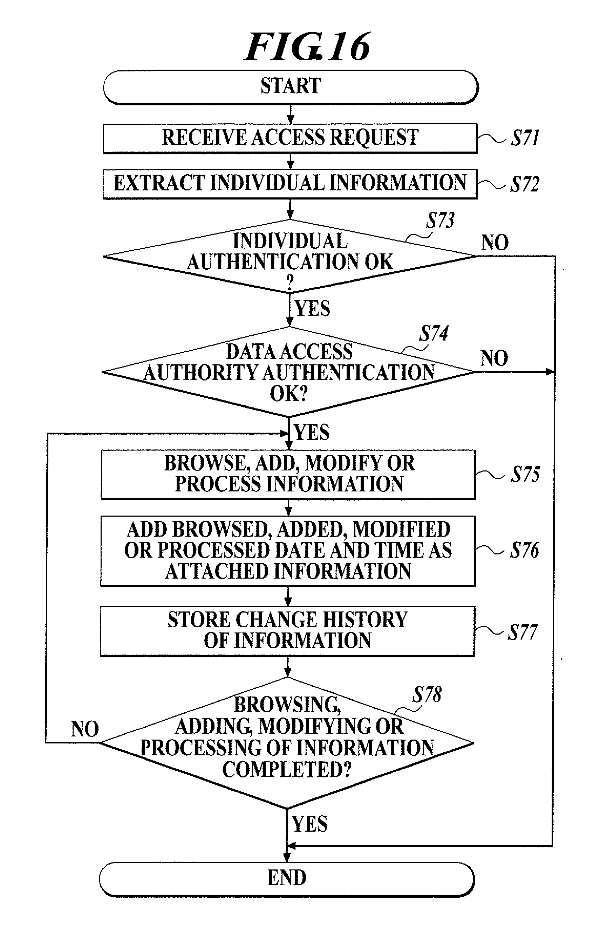

The control section 11 performs an information store process 2 (see FIG. 9) and an information usage process 6 (see FIG. 16) described later as processes characteristic of the sixth embodiment. The information store process 2 is the same as that in the above third embodiment, so that descriptions of it will be omitted.

FIG. 16 is a flow chart showing the information usage process 6 performed by the control section 11. As shown in FIG. 16, when the control section 11 receives an access request via the individual verification section 12 (step S71), the control section 11 makes the individual verification section 12 extract personal information (step S72) and perform personal authentication on an operator who is operating one of the operation terminals A to E which sent the access request. When personal authentication is succeeded (step S73; YES), then the control section 11 makes the data access authority authentication section 13 judge whether medical information access to which was requested is accessible or not (step S74).

When the medical information access to which was requested is accessible (step S74; YES), then the control section 11 makes the operator browse, add, modify or process the medical information stored in the database 16 in accordance with the access request (step S75). The control section 11 makes the date and time providing section 18 obtain the date and time when the medical information was browsed, added, modified or processed and add it to the medical information as attached information (step S76). Then, the control section 11 makes the data falsification prevention section 17 correlate a history indicative of this change with the medical information and store it in the database 16 (step S77).

The control section 11 judges whether the adding, modifying or processing of the medical information is completed (step S78). When the changing of the medical information is completed (step S78; YES), then the control section 11 terminates the information usage process 6. When the change of the medical information is not completed (step S78; NO), then the control section 11 returns to step S75 to repeat the above process.

As described above, in the sixth embodiment, the management server 1f makes the date and time providing section 18 add the date and time when new medical information was stored in the database 16 to the medical information as attached information. Moreover, when the medical information stored in the database 16 is added, modified or processed, the management server 1f makes the data falsification prevention section 17 and the date and time providing section 18 correlate a history indicative of this change and the date and time when the medical information was changed with the medical information and store them.

As a result, a change in medical information can be checked on a time-series basis and a change in the state of a patient can be recognized accurately. This will improve the accuracy and efficiency of a doctor's diagnosis. Moreover, by specifying the date and time when medical information was added, modified or processed and a history indicative of this change, the act of subsequently falsifying the medical information stored in the database 16 will be prevented. The reliability of medical information therefore can be improved.

### [Seventh Embodiment]

Next, the seventh embodiment of the present invention will be described.

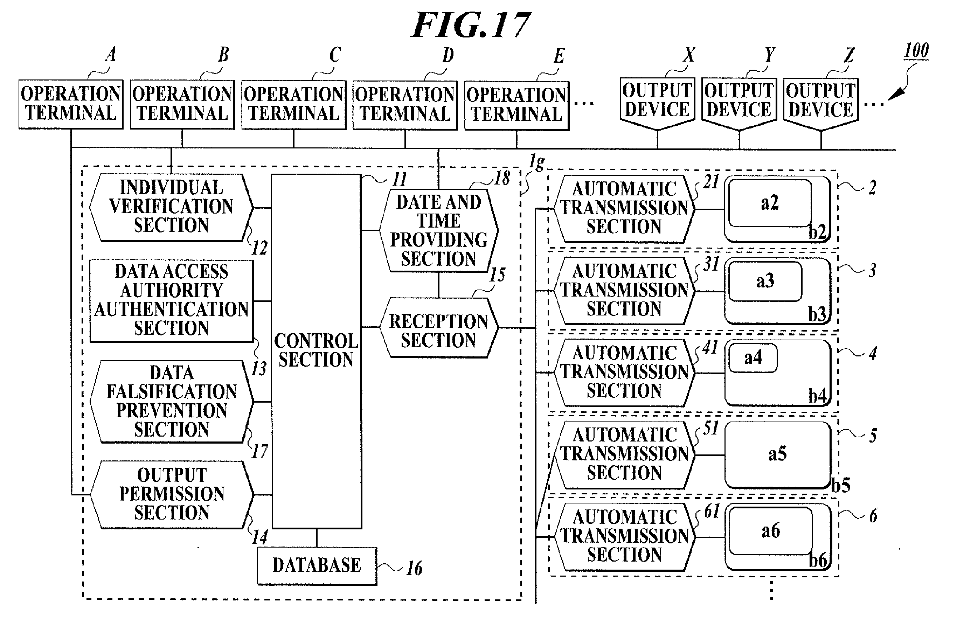

FIG. 17 is a block diagram showing the structure of principal portions of a management server 1g in the seventh embodiment. As shown in FIG. 17, the management server 1g comprises a control section 11, an individual verification section 12, a data access authority authentication section 13, an output permission section 14, a reception section 15, a database 16, a data falsification prevention section 17, a date and time providing section 18, and the like. That is, compared with the management server 1a in the above first embodiment, the management server 1g further comprises the data falsification prevention section 17 and the date and time providing section 18. The structure of the management server 1g is almost the same as that of the management server 1a in the above first embodiment, the management server 1b in the above second embodiment, or the management server 1c in the above third embodiment, so that detailed descriptions of the same structure will be omitted. Hereinafter, operation characteristic of the seventh embodiment will be described.

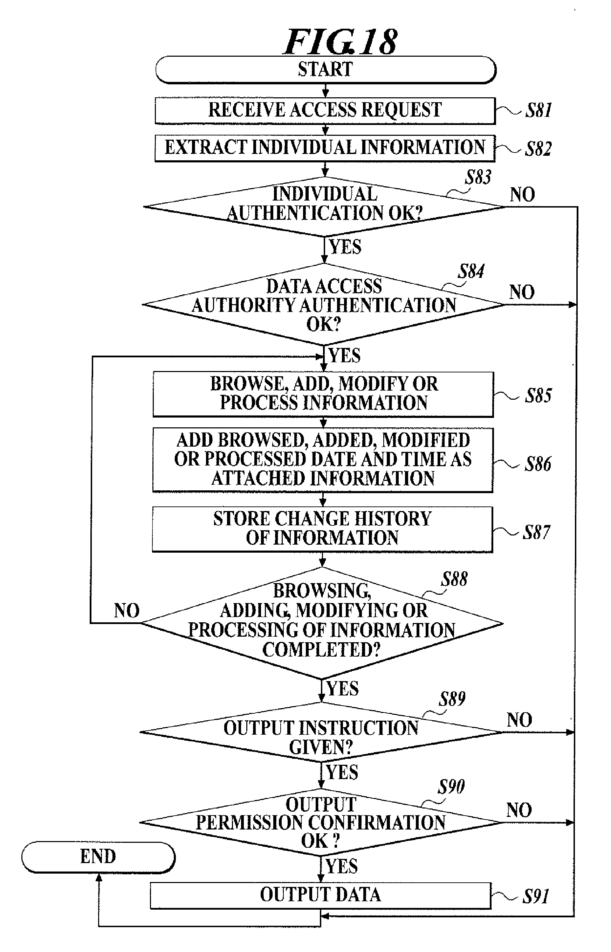

The control section 11 performs an information store process 2 (see FIG. 9) and an information usage process 7 (see FIG. 18) described later as processes characteristic of the seventh embodiment. The information store process 2 is the same as that in the above third embodiment, so that descriptions of it will be omitted.

FIG. 18 is a flow chart showing the information usage process 7 performed by the control section 11. As shown in FIG. 18, when the control section 11 receives an access request via the individual verification section 12 (step S81), the control section 11 makes the individual verification section 12 extract personal information (step S82) and perform personal authentication on an operator who is operating one of the operation terminals A to E which sent the access request. When personal authentication is succeeded (step S83; YES), then the control section 11 makes the data access authority authentication section 13 judge whether the medical information access to which was requested is accessible or not (step S84).

When the medical information access to which was requested is accessible (step S84; YES), then the control section 11 makes the operator browse, add, modify or process the medical information stored in the database 16 in accordance with the access request (step S85). The control section 11 makes the date and time providing section 18 obtain the date and time when the medical information was browsed, added, modified or processed and add it to the medical information 33 attached information (step S86). Then the control section 11 makes the data falsification prevention section 17 correlate a history indicative of this change with the medical information and store it (step S87).

The control section 11 judges whether the adding, modifying or processing of the medical information is completed (step S88). When the change of the medical information is completed (step S88; YES), then the control section 11 judges whether instructions to output the medical information have been inputted from the operation terminals A to E or not (step S89). When the instructions to output the medical information have been inputted from the operation terminals A to E (step S89; YES), then the control section 11 makes the output permission section 14 judge whether the specified medical information can be outputted (step S90). When the medical information can be outputted (step S90; YES), then the control section 11 makes the output devices X to Z output the medical information to the output permission section 14 (step S91), and terminates the information usage process 7.

As described above, in the seventh embodiment, the management server 1g makes the date and time providing section 18 add the date and time when new medical information was stored in the database 16 to the medical information as attached information. Moreover, when medical information stored in the database 16 is added, modified or processed, the management server 1g makes the data falsification prevention section 17 and the date and time providing section 18 correlate a history indicative of this change and the date and time when the medical information was changed with the medical information and store them. In addition, the management server 1g makes the output permission section 14 judge whether the specified medical information can be outputted. When permission to output the medical information is given, then the management server 1g makes the output devices X to Z output it.

This prevents a fraudulent activity, such as subsequently browsing, adding, modifying or processing medical information stored in the database 16 in the management server 1g, outflow of personal information relating to a patient to the outside, and copying of medical information to another record medium. As a result, the security of information management can be enhanced. Furthermore, the date and time when new medical information was sent or stored, or the date and time when medical information was updated is stored, so that a change in medical information becomes clear and a change in the state of a patient can be realized easily. This will improve the accuracy and efficiency of a doctor's diagnosis.

The above first to seventh embodiments are examples of the medical information management system according to the present invention, and the scope of the present invention is not limited to these embodiments.

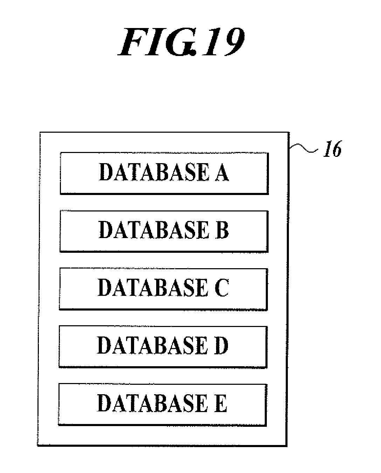

For example, the database 16 included in each of the management servers 1a to 1g may be formed as a single hard disk, or may be the ones having a plurality of volumes or partitions in a hard disk. Furthermore, the servers may be the ones having a plurality of hard disks connected in parallel, or a plurality of volumes or partitions in each of a plurality of connected hard disks. As shown in FIG. 19, it is preferable that the database 16 should include a plurality of databases A to E. By doing so, necessary information can be sent or received in a short period of time, or restoration or backup can be performed easily in the case of a hard disk crash. In this case, the control section 11 will select and obtain necessary medical information from the databases A to E in response to an access request sent from one of the operation terminals A to E.

In the medical information management system 200, for example, the network on which the management server 1, the medical institution DBs a to g, the operation terminals a1, b1, c1, d1, e1, f1, and g1, and the operation terminals α to ε is preferably built by dedicated lines from the viewpoint of security. However, when it is difficult to use a dedicated line, such as if a hospital, being the nucleus of a locality, and a family doctor's office or the like are connected, information to be sent or received preferably is encoded to prevent leakage of the information. In this case, each of the management server 1, the medical institution DBs a to g, the operation terminals a1, b1, c1, d1, e1, f1, and g1, and the operation terminals α to ε comprises an encoding section (not shown) for encoding information to be sent and a decoding section (not shown) for decoding information received. At this time, for example, a secret key cryptosystem, a public key cryptosystem, or the like will be used. Besides medical information, information to be encoded preferably includes instructions relating to operation, personal information relating to an operator, and the like included in the above access request.

It is needless to say that the structure and operation of the details of the components in each of the medical information management systems in the first to seventh embodiments can be modified properly in a range within the scope of the present invention.

### Industrial Applicability

In accordance with the present invention, in the medical information management system in which the medical information is managed integrally, verification of the operator for operating the medical information is performed, and moreover, the medical information to be operated by the verified operator is authenticated whether or not it is the information capable of being operated. Thereby, the medical information can be prevented from being operated improperly, and the security of information management can be improved. Further, when outputting the medical information, since it is outputted after authenticating whether the medical information instructed to be outputted is the information capable of being outputted, it becomes possible to prevent the important medical information from being leaked to outsiders.

Further, when the operation of adding, modifying, processing or the like is performed to the medical information, since the change history is stored by being correlated with the medical information while maintaining the original medical information, it becomes possible to prevent the important medical information from being rewritten mistakenly or to prevent the medical information from being falsified improperly. Further, since the stored date and time or the changed date and time of the medical information is provided as attached information, an effect of inhibiting actions of operating the information improperly can be obtained.

## Claims

1. A medical information management system comprising:
a plurality of database devices for storing medical information in a database;
a management device for managing the medical information integrally; and
an operation terminal for operating at least one of the database devices and the management device, each of the database devices comprising an automatic transmission section for extracting necessary information from the medical information stored in the database and sending the necessary information to the management device,
the management device comprising:
a reception section for receiving the medical information sent from the automatic transmission section;
a storage section for storing integrally the medical information received by the reception section on a basis of predetermined information included in the medical information;
an individual verification section for verifying whether or not an operator of the operation terminal is an individual registered beforehand; and
a data access authentication section for judging whether or not medical information instructed to be operated by the operation terminal is information capable of being operated.

2. The system of claim 1, wherein the management device further comprises a control section for performing at least one of browsing, adding, modifying and processing of the medical information in a case that the medical information instructed to be operated is judged as the information capable of being operated by the data access authentication section on the basis of operation instruction from the operator verified by the individual verification section.

3. The system of claim 1 or claim 2, comprising:
an output device for recording the medical information in a recording medium as electronic information or outputting the medical information in a printing medium as a hardcopy,
wherein the management device further comprises an output permission section for judging whether or not medical information instructed to be outputted by the operation terminal is information capable of being outputted to the output device and for outputting the medical information to the output device in case that the medical information is the information capable of being outputted.

4. The system of claim 2, wherein the management device further comprises a data falsification prevention section for storing change history in the storage section by making the change history correlate with the medical information in case that the adding, modifying or processing is performed to the medical information store in the storage section by the control section.

5. The system of claim 2 or claim 4, wherein the management device further comprises a date and time providing section for adding a date and time when the medical information is operated as attached information in case that the browsing, adding, modifying or processing is performed to the medical information stored in the storage section by the control section.

6. The system of claim 5, wherein the date and time providing section adds the date and time when the medical information sent by the automatic transmission section is received by the reception section or the date and time when the medical information sent by the automatic transmission section is stored in the storage section to the medical information as the attached information.

7. The system of claim 1, wherein the automatic transmission section detects difference between medical information to be sent and medical information sent in past, extracts medical information corresponding to the difference, and sends the extracted medical information to the management device as the necessary information.

8. The system of claim 1, wherein the medical information sent by the automatic transmission section includes a creation date and time when the medical information is created, and
the management device further comprises a storage control section for comparing a creation date and time included in the medical information received by the reception section with a creation date and time included in the medical information stored in the storage section, and for storing the medical information received by the reception section, which includes the creation date and time different from the creation date and time included in the medical information stored in the storage section.

9. The system of claim 1, wherein the individual verification section verifies the individual according to at least one kind of individual information selected from a password, an ID card, a fingerprint, a palm print, a voiceprint, a face, a signature handwriting, an iris pattern, an eyeground pattern and a vein pattern.

10. The system of claim 9, wherein the individual information is at least one of the fingerprint, the palm print, the voiceprint, the face, the iris pattern, the eyeground pattern and the vein pattern.

11. The system of claim 9, wherein the individual verification section verifies the individual in combination with the individual information of the password or the ID card and at least one of the fingerprint, the voiceprint, the iris pattern, and the vein pattern.

12. The system of claim 2, wherein the medical information for being browsed, added, modified or processed by the control section is at least one kind of data selected from character data, on-off data, static image data and dynamic image data.

13. The system of claim 1, wherein the operation terminal comprises an input member for inputting the operation instruction, and
the input member is at least one kind of member selected from a pen, a keyboard, a mouse and voice.

14. The system of any one of claims 1 to 8 and 12, wherein the medical information includes at least one kind of data selected from clinical examination management system data, radiation department system data, hospital information system data, electronic chart system data, case management system data, medicine history management system data, medicine document data, nursing-care insurance system data, medical-related document data.

15. The system of any one of claims 1 to 8, 12 and 14, wherein the medical information sent by the automatic transmission section includes at least one kind of data selected from clinical examination management system data, radiation department system data, hospital information system data, electronic chart system data, case management system data, medicine history management system data, medicine document data, nursing-care insurance system data, medical-related document data.

16. The system of claim 1, wherein the management device and the operation terminal are connected via a network, and the network comprises a dedicated line for connecting the management device and the operation terminal.

17. The system of claim 1 or claim 16, wherein the management device further comprises:
an encryption transmission section for encrypting and sending information sending to the operation terminal; and
a decryption reception section for receiving and decrypting information sent from the operation terminal, and
the operation terminal comprises:
an encryption transmission section for encrypting and sending the information sending to the management device; and
a decryption reception section for receiving and decrypting the information sent from the management device.

18. The system of claim 2, wherein the storage section of the management device comprises a plurality of databases for storing information in accordance with a kind of the medical information, and
the control section reads out the corresponding medical information from the plurality of the databases in accordance with the operation instruction from the operation terminal, and controls at least one operation of the browsing, adding, modifying and processing.

19. A medical information management system comprising:
at least one database for storing medical information; and
a management section for managing access to the database,
wherein the management section stores a biological discrimination pattern peculiar to an operator who accesses the database by making the biological discrimination pattern correlate with ID information set to the operator, obtains a biological discrimination pattern and ID information from an arbitrary operator when an access is required to the database from the arbitrary operator, and judges whether or not to approve the access by comparing the obtained biological discrimination pattern and the ID information with the biological discrimination pattern and the ID information correlated and stored beforehand.

20. The system of claim 19, wherein the peculiar biological discrimination pattern is at least one of a fingerprint, a palm print, a voiceprint, a face, an iris pattern, a eyeground pattern and a vein pattern.
